# EUROPÄISCHE PATENTANMELDUNG

(11) **EP 1 393 801 A1**
(43) Veröffentlichungstag der Anmeldung: **03.03.2004**
(21) Anmeldenummer: 03019717.2
(22) Anmeldetag: 29.08.2003
(51) Int. Cl.: B01J 23/66, C07D 301/10

(54) **Silberkatalysator für die Epoxidation von Propylen**

(30) Priorität: 30.08.2002 DE 10240128
(71) Anmelder: BASF AKTIENGESELLSCHAFT, 67056 Ludwigshafen (DE)
(72) Erfinder: Borchert, Holger, Dr., 67591 Offstein (DE); Schlitter, Stephan, Dr., 67117 Limburgerhof (DE); Muhler, Martin, Prof. Dr., 44799 Bochum (DE); Busser, Wilmer, Dr., 45468 Mühlheim an der Ruhr (DE)
(74) Vertreter: Isenbruck, Günter, Dr.

(57) **Zusammenfassung**

Verfahren zur Herstellung von Propylenoxid durch Gasphasenoxidation eines Gemisches, umfassend Propylen und Sauerstoff, bei einer Temperatur von 100 bis 300 °C und einem Druck von 1 bis 30 bar an einem Katalysator, dadurch gekennzeichnet, dass der Katalysator
(i) Silber,
(ii) mindestens eine Erdalkalimetallverbindung,
(iii) eine Kaliumverbindung oder ein Gemisch einer Kaliumverbindung mit mindestens einer weiteren Verbindung, wobei das Gemisch einen Schmelzpunkt unterhalb 340 °C aufweist, und
(iv) eine Kombination einer Cäsiumverbindung mit wenigstens einer Verbindung eines Elementes, ausgewählt aus der Gruppe Molybdän, Rhenium und Wolfram,
umfasst.

## Beschreibung

Die vorliegende Erfindung betrifft ein Verfahren zur Direktoxidation von Propylen mit molekularem Sauerstoff zu Propylenoxid unter Verwendung eines neuen Silberkatalysators. Dieser enthält eine Erdalkalimetallverbindung, als Promotor wenigstens eine Kaliumverbindung oder ein Gemisch einer Kaliumverbindung mit einer weiteren Verbindung, wobei das Gemisch einen Schmelzpunkt unterhalb 340 °C aufweist, und eine Kombination einer Cäsiumverbindung mit einer Verbindung ausgewählt aus der Gruppe der Elemente Molybdän, Rhenium und Wolfram. Die Erfindung betrifft auch den Katalysator an sich, an dem die Umsetzung von Propylen mit Sauerstoff zu Propylenoxid stattfindet, sowie ein Verfahren zur Herstellung des Katalysators.

Oxirane können sowohl durch Umsetzung von Alkenen mit Hydroperoxiden als auch durch Direktoxidation in der Gasphase mit molekularem Sauerstoff hergestellt werden. Beide Reaktionstypen benötigen im Allgemeinen die Mitwirkung von Katalysatoren. Während die Direktoxidation von Ethen zu Ethylenoxid mit hoher Selektivität und hohem Umsatz und daher hoher Ausbeute relativ einfach durchgeführt werden kann, ist dieses Verfahren zur Umsetzung der höheren Olefine weniger geeignet. Hier tritt als störende Konkurrenzreaktion zur Alkenoxidbildung die völlige Oxidation des Alkens unter Abbau zu Kohlendioxid auf.

Beispielsweise zeigt die Direktoxidation von Propylen zu Propylenoxid, das ein wichtiges Zwischenprodukt für organische Synthesen darstellt, dieses ungünstige Verhalten. Es hat daher nicht an Versuchen gefehlt, geeignete Katalysatoren zu entwickeln, mit denen die Direktoxidation von Propylen zu Propylenoxid unter hoher Selektivität und Ausbeute durchgeführt werden kann.

Typischerweise werden für die Direktoxidation von Propylen Katalysatoren verwendet, die metallisches oder ionisches Silber und Promotoren und Aktivatoren enthalten. Meistens sind die Katalysatoren auf ein Trägermaterial aufgezogen.

Beispielsweise ist ein geträgerter Katalysator bekannt, der als Träger ein Erdalkalimetallcarbonat, metallisches Silber als Katalysator sowie Kaliumnitrat als Promotor enthält (CA 1,282,772).

Neben den Erdalkalimetallverbindungen wurden als Trägermaterialien auch bereits Erdalkalimetalltitanate, dreibasisches Calciumphosphat, Calciummolybdat und Calciumfluorid vorgeschlagen (WO 97/34693).

Auch ist bereits bekannt, als Promotor zusätzlich eine Goldverbindung zur Erhöhung der Selektivität der Propylenoxidbildung mit zu verwenden (US 5,703,254).

Als weitere Promotoren wurden auch bereits Molybdän- (US 5,686,380), Wolfram- (WO 98/52931), Rhenium- (US 5,864,047) und anorganische Chloridverbindungen (US 5,965,480) verwendet. Auch Promotoren auf Magnesiumbasis können zusammen mit Eisen/Cobalt-Promotoren eingesetzt werden (WO 99/06385).

Im Zusammenhang mit vorstehend erwähnten Molybdänverbindungen wurden in der US 5,686,380 als Promotoren bereits Ammonium-, Kalium-, Natrium- und Cäsiumverbindungen mit Wolfram genannt. Wie den Beispielen der US 5,686,380 zu entnehmen ist, offenbaren diese jedoch nur die Verwendung von Ammoniummolybdat. Eingehender wurde dagegen die Eignung von Alkaliverbindungen als Promotoren in der später veröffentlichten US 5,864,047 untersucht. Während für Kaliumverbindungen positive Ergebnisse erhalten wurden, konnte die Eignung der anderen Alkalimetallverbindungen nicht bestätigt werden. Insbesondere Verbindungen mit Cäsium, die ansonsten wirkungsvoll in die Direktoxidation von Ethen eingesetzt werden können, erwiesen sich für die Direktoxidation von Propylen überraschenderweise als wenig geeignet, da sie nicht in der Lage waren, die katalytische Aktivität des Katalysatorsystems merklich zu verbessern.

Ferner zeigen die bisher vorliegenden Ergebnisse, dass eine hohe Selektivität bezüglich der Propylenoxidbildung nur dann erreicht werden kann, wenn der Anteil an Propylen im zu oxidierenden Gasgemisch relativ niedrig ist. Diese hohe Selektivität geht aber mit einer niedrigen Umsatzrate von Propylen zu Propylenoxid und damit mit einer niedrigen Ausbeute einher, und umgekehrt. Beispielsweise konnte ein Gasstrom, der 5 Vol.-% Propylen enthielt, mit einer Selektivität von immerhin 61 % oxidiert werden, wobei allerdings lediglich ein Umsatz von ca. 4,5 % erreicht wurde. Dies entspricht einer berechneten Ausbeute an Propylenoxid von 2,75 % (Produkt aus Umsatz und Selektivität) bzw. von ca. 0,14 Vol.-% an Propylenoxid im Abgasstrom (US 5,686,380; Beispiel 2). Ein Gasstrom, der 10 Vol.-% Propylen enthielt, konnte mit einem relativ hohen Umsatz von 14 %, aber lediglich einer Selektivität von 37 % umgesetzt werden, was einer Ausbeute an Propylenoxid im Abgasstrom von ca. 0,53 Vol.-% entspricht. Bei einem Umsatz von 7 % und einer Selektivität von 57 % sank die Ausbeute an Propylenoxid auf ca. 0,4 Vol.-% im Abgasstrom ab (US 5,864,047; Tabelle 2 in Beispiel 3).

Es war daher die Aufgabe gestellt, einen Katalysator zur Verfügung zu stellen, der die Direktoxidation von Propylen zu Propylenoxid mit einer guten Selektivität und einer guten Konversionsrate und damit einer guten Ausbeute ermöglicht.

Diese Aufgabe konnte gelöst werden mit einem geträgerten Silberkatalysator, der mindestens eine Erdalkalimetallverbindung, als Promotor wenigstens eine Kaliumverbindung oder ein Gemisch einer Kaliumverbindung mit einer weiteren Verbindung, wobei das Gemisch einen Schmelzpunkt unterhalb 340 °C aufweist, und eine Kombination einer Cäsiumverbindung mit einer Verbindung, ausgewählt aus der Gruppe der Elemente Molybdän, Rhenium und Wolfram, enthält.

Die vorliegende Erfindung betrifft somit ein Verfahren zur Herstellung von Propylenoxid durch Gasphasenoxidation eines Gemisches, umfassend Propylen und Sauerstoff, bei einer Temperatur von 100 bis 300 °C und einem Druck von 1 bis 30 bar an einem Katalysator, dadurch gekennzeichnet, dass der Katalysator als Komponenten
(i) Silber,
(ii) mindestens eine Erdalkalimetallverbindung,
(iii) eine Kaliumverbindung oder ein Gemisch einer Kaliumverbindung mit mindestens einer weiteren Verbindung, wobei das Gemisch einen Schmelzpunkt unterhalb 340 °C aufweist, und
(iv) eine Kombination einer Cäsiumverbindung mit wenigstens einer Verbindung eines Element, ausgewählt aus der Gruppe Molybdän, Rhenium und Wolfram,
umfasst, sowie
einen Katalysator zur Durchführung des erfindungsgemäßen Verfahrens der Direktoxidation von Propylen mit Sauerstoff, dadurch gekennzeichnet, dass der Katalysator als Komponenten
(i) Silber,
(ii) mindestens eine Erdalkalimetallverbindung,
(iii) eine Kaliumverbindung oder ein Gemisch einer Kaliumverbindung mit mindestens einer weiteren Verbindung, wobei das Gemisch einen Schmelzpunkt unterhalb 340 °C aufweist, und
(iv) eine Kombination einer Cäsiumverbindung mit wenigstens einer Verbindung mit einem Element, ausgewählt aus der Gruppe Molybdän, Rhenium und Wolfram,
umfasst.

Mit dem neuen Katalysator lassen sich bei einer Ausbeute an Wertstoff zwischen 1,3 und 0,8 Vol.-% im Abgasstrom bei Selektivitäten, die zwischen 54 und 62 % liegen, Umsatzraten zwischen 25 und 20 % erzielen. Ferner war es aufgrund des im Stand der Technik beschriebenen Vorurteils nicht vorhersehbar, dass diese guten Ergebnisse mit einem Katalysator erreicht werden können, der als Promotor Cäsiumverbindungen enthält. Somit ist dieses Ergebnis unerwartet und als außerordentlich überraschend zu bezeichnen.

Des Weiteren betrifft die Erfindung auch ein Verfahren zur Herstellung des Katalysators.

Silber (i) kann sowohl in metallischer Form als auch in ionischer Form verwendet werden. Beispielsweise ist für die Herstellung des Katalysators die Verwendung von Silberoxid, Silberoxalat, Silbernitrat oder Silbercarbonat möglich.

Als Erdalkalimetallverbindung (ii) können die im Stand der Technik, etwa in US 5,763,630 oder US 5,864,047 beschriebenen Verbindungen eingesetzt werden.

Dies sind vorzugsweise anorganische Verbindungen, die ein oder mehrere Erdalkalimetalle, insbesondere Calcium, Strontium, Magnesium oder Barium enthalten, wobei Calcium, Strontium und Barium bevorzugt sind. In Abhängigkeit vom gewählten Erdalkalimetall kann die Erdalkalimetallverbindung ein Carbonat, Titanat, Phosphat, Aluminat, Molybdat, Fluorid oder eine Kombination aus zwei oder mehr davon enthalten. Insbesondere können als Erdalkalimetallverbindungen Erdalkalimetallcarbonate, Erdalkalimetalltitanate, Calciumphosphat, insbesondere dreibasisches Calcium-phosphat, Magnesiumaluminat, Calciummolybdat, Calciumfluorid oder Gemische aus zwei oder mehr dieser Verbindungen eingesetzt werden. Das dreibasische Calciumphosphat kann durch die empirische Formel Ca₁₀(OH)₂(PO₄)₆ charakterisiert werden, das Calciummolybdat durch die Formel CaMoO₄. Die Erdalkalimetalltitanate umfassen Titanate der allgemeinen Formeln MTiO₃, M₂TiO₄ und MTi₂O₅, wobei M vorzugsweise für Barium, Strontium, Calcium oder Magnesium steht, insbesondere jedoch für Calcium oder Strontium.

Als Erdalkalimetallverbindungen sind besonders Carbonate bevorzugt, insbesondere Calciumcarbonat, Strontiumcarbonat oder Bariumcarbonat. Hierbei sind auch Gemische aus Calciumcarbonat mit Strontiumcarbonat oder Calciumcarbonat mit Bariumcarbonat möglich. Insbesondere bevorzugt ist Calciumcarbonat.

Demzufolge ist eine bevorzugte Ausführungsform des erfindungsgemäßen Verfahrens dadurch gekennzeichnet, dass die Erdalkalimetallverbindung ausgewählt wird aus der Gruppe bestehend aus Calcium-, Strontium-, Bariumcarbonat und einem Gemisch aus zwei oder mehr davon.

Die Erdalkalimetallverbindungen können dabei auch als Trägermaterialien eingesetzt werden.

Weitere geeignete Trägermaterialien, auf denen die Erdalkalimetallverbindung sowie die weiteren Komponenten des Katalysators aufgebracht sein oder auf diesen vorliegen können, sind vorzugsweise inerte Trägermaterialien wie beispielsweise Aluminiumoxid, Siliciumdioxid, Siliciumcarbid, Titandioxid und/oder Zirkoniumdioxid. Besonders bevorzugt ist als inertes Trägermaterial Aluminiumoxid, wobei es sich um jedes Aluminiumoxid handeln kann. Besonders bevorzugt handelt es sich um ein α-Aluminiumoxid oder ein hoch geglühtes Aluminiumoxid mit einer spezifischen Oberfläche von < 10 m²/g, gemessen nach DIN 66 131.

Demgemäß beschreibt die vorliegende Erfindung auch einen wie oben beschriebenen Katalysator, wobei der Katalysator ein geträgerter Katalysator ist.

Bei den unter (iii) und (iv) aufgeführten Verbindungen handelt es sich um Promotoren, die die Eigenschaften des Katalysatorsystems bezüglich der Propylenoxidbildung günstig beeinflussen.

Bei der unter (iii) aufgeführten Kaliumverbindung des Promotors kann es sich um jede beliebige Kaliumverbindung handeln. Bevorzugt handelt es sich hierbei jedoch um Kaliumnitrat oder eine Kaliumverbindung, die unter den Epoxidationsbedingungen Kaliumnitrat bildet oder bilden kann.

Vorzugsweise enthält die Kaliumverbindung noch mindestens eine weitere Verbindung, wobei dann das resultierende Gemisch einen Schmelzpunkt unterhalb 340 °C, vorzugsweise unterhalb 320 °C, insbesondere unterhalb 300 °C aufweist. Hierbei handelt es sich vorzugsweise um Schmelzpunktwerte, die nach thermometrischen Methoden bestimmt werden (Römpp Chemie Lexikon, Georg Thieme Verlag, Stuttgart, New York, 9. Auflage, S. 4569).

Diese Promotoren-Gemische weisen somit Schmelzpunkte auf, die bereits im Temperaturbereich der Umsetzung des Propylens mit Sauerstoff liegen. Diese Maßnahme hat sich als besonders wirksam für das Erreichen einer hohen Selektivität bei gleichzeitig hoher Ausbeute erwiesen.

Vorzugsweise enthält besagtes Gemisch neben der Kaliumverbindung mindestens eine weitere Verbindung, die ein von Kalium verschiedenes Metall umfasst, ausgewählt aus Alkalimetallen, Erdalkalimetallen, Blei, Silber und Thallium.

Geeignete Promotorgemische, die im oben definierten Schmelzpunktbereich liegen, können durch routinemäßige Versuche bestimmt und/oder Phasendiagrammen entnommen werden, wie sie beispielsweise in vorstehend genannter Literaturstelle beschrieben sind. Das Promotorgemisch enthält neben der Kaliumverbindung somit mindestens eine weitere Verbindung, wobei sich hierfür grundsätzlich jede Verbindung eignet, die kein Kalium enthält und die sich mit der Kaliumverbindung zu einem Gemisch verbindet, das einen Schmelzpunkt im definierten Bereich aufweist. Hierbei sind solche Verbindungen bevorzugt, die unter den Reaktionsbedingungen chemisch weitestgehend stabil sind und keine unerwünschten Reaktionen katalysieren.

Geeignete Verbindungen sind insbesondere Natriumnitrat, Natriumnitrit, Lithium-, Blei-, Barium-, Silber-, Cäsium-, Rubidium- und Thalliumnitrat, wobei Natriumnitrat, Natriumnitrit und Lithiumnitrat besonders bevorzugt sind. Neben binären Gemischen sind auch Gemische der Kaliumverbindung mit zwei oder mehr der oben genannten Verbindungen möglich.

Besonders bevorzugt ist als Promotorgemisch ein Gemisch aus Kaliumnitrat und einer Verbindung, ausgewählt aus der Gruppe, bestehend aus Natriumnitrat, Natriumnitrit, Lithium-, Blei-, Barium-, Silber-, Cäsium-, Rubidium- und Thalliumnitrat.

Demzufolge ist eine besonders bevorzugte Ausführungsform des erfindungsgemäßen Katalysators dadurch gekennzeichnet, dass die Kaliumverbindung Kaliumnitrat ist und die mindestens eine weitere Verbindung aus der Gruppe, bestehend aus Natriumnitrat, Natriumnitrit, Lithiumnitrat, Bleinitrat, Bariumnitrat, Silbemitrat, Cäsiumnitrat, Rubidiumnitrat und Thalliumnitrat, ausgewählt ist.

Weiterhin enthält der Katalysator eine Promotorkombination (iv) aus einer Cäsiumverbindung mit wenigstens einer Verbindung der Elemente Molybdän, Rhenium und/oder Wolfram.

Als Cäsiumverbindung kann jede beliebige Cäsiumverbindung eingesetzt werden. Vorzugsweise wird jedoch Cäsiumnitrat, Cäsiumhydroxid, Cäsiumchlorid, Cäsiumfluorid, Cäsiumsulfat, Cäsiumoxalat oder Cäsiumacetat eingesetzt. Ebenso sind zwei oder mehr geeignete Cäsiumverbindungen, insbesondere zwei oder mehr der als bevorzugt genannten Cäsiumverbindungen einsetzbar. Besonders bevorzugt sind Cäsiumnitrat und Cäsiumhydroxid.

Bei den Verbindungen der Metalle Rhenium, Molybdän und Wolfram kann jede beliebige Verbindung dieser Elemente eingesetzt werden. Bevorzugt sind Ammoniumpherrhenat, Rhenium(III)-chlorid, Rhenium(V)-chlorid, Rhenium(VI)-fluorid, Rhenium(VI)-oxid, Rhenium(VII)-oxid, Ammoniumwolframat, Ammoniummetawolframat, Wolfram(IV)-chlorid, Wolfram(VI)-chlorid, Wolframoxytetrachlorid, Wolframsäure, Ammoniummolybdat, Molybdän(III)-chlorid, Molybdän(V)-chlorid und Molybdän(VI)-chlorid-oxid. Ebenso sind zwei oder mehr geeignete Rhenium-, Molybdän- und/oder Wolframverbindungen, insbesondere zwei oder mehr der als bevorzugt genannten Verbindungen einsetzbar.

Insbesondere sind Ammoniumperrhenat, Ammoniumwolframat und Ammoniummolybdat bevorzugt.

Somit ist eine besonders bevorzugte Ausführungsform des erfindungsgemäßen Verfahrens dadurch gekennzeichnet, dass die Cäsiumverbindung Cäsiumnitrat oder Cäsiumhydroxid ist und kombiniert wird mit einer oder mehreren Verbindung, ausgewählt aus der Gruppe bestehend aus Ammoniumperrhenat, Ammoniumwolframat und Ammoniummolybdat.

Der erfindungsgemäße Katalysator enthält vorzugsweise die Komponenten (i) bis (iv) in den folgenden Mengen, wobei die Gesamtmenge der Komponenten 100 Gew.-% ergibt:
(i) 1 bis 50 Gew.-%, weiter bevorzugt 5 bis 35 Gew.-%, insbesondere bevorzugt 10 bis 25 Gew.-% Silber;
(ii) 1 bis 50 Gew.-%, weiter bevorzugt 5 bis 30 Gew.-%, insbesondere bevorzugt 10 bis 25 Gew.-% Erdalkalimetallverbindung;
(iii) 0 bis 95 Gew.-%, weiter bevorzugt 20 bis 90 Gew.-%, insbesondere bevorzugt 30 bis 80 Gew.-% Trägermaterial;
   0,1 bis 20 Gew.-%, weiter bevorzugt 1 bis 15 Gew.-%, insbesondere bevorzugt 2 bis 10 Gew.-% Kaliumverbindung;
   0,01 bis 20 Gew.-%, weiter bevorzugt 0,05 bis 15 Gew.-%, insbesondere bevorzugt 0,1 bis 10 Gew.-% der weiteren Verbindung des Gemisches mit der Kaliumverbindung;
(iv) 10 bis 10.000 ppm, weiter bevorzugt 50 bis 5.000 ppm, insbesondere bevorzugt 50 bis 1.000 ppm Cäsium;
   0,01 bis 10 Gew.-%, weiter bevorzugt 0,05 bis 5 Gew.-%, insbesondere bevorzugt 0,1 bis 2 Gew.-% einer oder mehrerer Verbindungen aus der Gruppe der Elemente Rhenium, Molybdän und Wolfram.

Der Katalysator kann darüber hinaus weitere Promotoren oder Bestandteile enthalten, insbesondere dann, wenn diese die Selektivität und/oder Aktivität steigern. Bevorzugt sind hierbei die Promotoren Schwefel, Chrom, Silizium, Titan, Zirkonium, Phosphor, Bor, Selen und Hafnium. Der Gehalt an solchen weiteren Bestandteilen und/oder Promotoren beträgt vorzugsweise 0 bis 5 Gew.-%., insbesondere 0 bis 2 Gew.-% und ganz besonders bevorzugt 0,01 bis 2 Gew.-%.

Die Epoxidierung von Propylen wird als Gasphasenreaktion in Gegenwart des Katalysators und eines Ausgangsgemisches aus Propylen und einem Sauerstoff enthaltendem Gas durchgeführt. Es ist aber auch bekannt, dass Zusätze von Halogen oder Halogenverbindungen zu diesem Ausgangsgemisch sich günstig auf die Propylenoxidbildung auswirken können. Vorzugsweise werden deshalb solche Ausgangsgemische eingesetzt, die Halogen oder Halogenverbindungen enthalten.

Als Sauerstoff enthaltendes Gas kann zweckmäßigerweise Luft, mit Sauerstoff angereicherte Luft oder reiner Sauerstoff verwendet werden.

Bei dem Halogen handelt es sich vorzugsweise um Chlor, während bevorzugte Halogenverbindungen ein organisches Halogenid, beispielsweise Ethylchlorid, Methylchlorid, Vinylchlorid oder Ethylendichlorid, umfassen.

Ferner kann das Ausgangsgemisch weiterhin eine Stickstoffverbindung, beispielsweise Stickstoffmonoxid (NO), Stickstoffdioxid (NO₂), Distickstofftetroxid (N₂O₄) und/oder Distickstoffoxid (N₂O) enthalten. Aus dieser Gruppe von Stickstoffoxiden können Stickstoffmonoxid und Stickstoffdioxid besonders vorteilhaft mitverwendet werden.

Darüber hinaus können im Ausgangsgemisch beispielsweise Stickstoff, Methan, Ethan, Propan, Butan, Wasserdampf und/oder Kohlendioxid vorliegen.

Die Epoxidierung wird vorzugsweise in einem Temperaturbereich von 100 bis 300 °C, insbesondere 200 bis 280 °C durchgeführt. Der Druck liegt dabei vorzugsweise im Bereich von 1 bis 30 bar, insbesondere 1 bis 20 bar.

Vorzugsweise werden Ausgangsgemische eingesetzt, die die vorstehend genannten Verbindungen oder Elemente in folgenden Anteilen enthalten, jeweils bezogen auf 100 Vol.-% Ausgangsgemisch:
1 bis 30 Vol.-% Propylen, 5 bis 50 Vol.-% Sauerstoff, 0 bis 1000 ppm Stickoxid(e), 0 bis 1000 ppm Halogen oder Halogenverbindungen. Zur Ergänzung auf 100 Vol.-% können Kohlendioxid, Wasserdampf und/oder unter den Bedingungen inertes Gas, zum Beispiel Stickstoff, Methan, Ethan und/oder Propan, verwendet werden.

Somit ist das erfindungsgemäße Verfahren auch dadurch gekennzeichnet, dass in die Gasphasenoxidation ein Gemisch eingesetzt wird, das 1 bis 30 Vol.-% Propylen, 5 bis 50 Vol.-% Sauerstoff, 0 bis 1000 ppm Stickoxide, 0 bis 1000 ppm Halogen oder Halogenverbindungen sowie weitere Verbindungen ausgewählt aus der Gruppe Kohlendioxid, Wasserdampf, Stickstoff, Ethan und Propan umfasst, wobei die Gesamtmenge aller eingesetzten Verbindungen 100 Vol.-% ergibt.

Die für die Epoxidation geeigneten Reaktoren sind bekannt. Beispielsweise können Reaktoren verwendet werden, in denen der Katalysator als heterogener Katalysator vorliegt. Solche Reaktoren sind beispielsweise Festbett-, Fließbett- oder Wirbelbett-Reaktoren, die beispielsweise als Rohrreaktoren ausgeführt sein können.

Die Herstellung des Katalysators unterliegt keinen besonderen Einschränkungen. Es eignen sich alle Verfahren, bei denen die Bestandteile des Katalysators in geeigneter Weise miteinander umgesetzt werden, wobei die Reihenfolge der Umsetzungsschritte keinen besonderen Beschränkungen unterworfen ist.

Vorzugsweise erfolgt die Herstellung des Katalysators so, dass das Silber und die Promotoren auf die Erdalkalimetallverbindung, die gegebenenfalls auf inertem Trägermaterial vorliegt, aufgebracht werden. Beispielsweise kann als inertes Trägermaterial Aluminiumoxid vorliegen, auf das die weiteren Komponenten, aus denen der Katalysator hergestellt werden soll, aufgebracht werden. Es können die Herstellungsverfahren verwendet werden, wie sie auch im Stand der Technik beschrieben sind, beispielsweise in der CA 1,282,772.

In einer bevorzugten Ausgestaltung des Herstellungsprozesses wird die Promotorkombination aus der Cäsiumverbindung und einer oder mehrerer Verbindungen aus der Gruppe der Elemente Rhenium, Molybdän und Wolfram (iv) zusammen mit dem Silber in Form eines löslichen Salzes oder Komplexes (i) und der Erdalkalimetallverbindung (ii), insbesondere Calciumcarbonat, auf einen inerten Aluminiumoxidträger aufgebracht. Der feuchte oder vorgetrocknete Träger wird anschließend calciniert, um die Silberverbindung in elementares Silber umzuwandeln.

Um eine Silberverbindung in löslicher Form zu erhalten, kann einer Silberverbindung wie Silber(I)-oxid, Silber(I)-nitrat oder Silber(I)-oxalat in geeigneter Weise ein Komplexierungsmittel, wie beispielsweise Ethanolamin, sec.-Butylamin, Oxalsäure und/oder Ethylendiamin, zugesetzt werden. Solche Komplexierungsmittel können auch gleichzeitig als Reduktionsmittel wirken. Zu dieser Lösung wird nun die Promotorenkombination aus einer in dieser Lösung löslichen Cäsiumverbindung und löslichen Verbindungen aus der Gruppe der Elemente Rhenium, Molybdän und/oder Wolfram gegeben. Auf den beschichteten Träger wird dann das Gemisch der Kaliumverbindung mit mindestens einer weiteren Verbindung, die kein Kalium enthält, aufgebracht. Der Kaliumpromotor bzw. das Promotorengemisch wird vorteilhafterweise als Lösung, insbesondere als wässerige Lösung beispielsweise durch Imprägnieren aufgebracht.

Für einen Katalysator, der nicht auf einem inerten Trägermaterial aufgebracht ist, hat sich ein Verfahren bewährt, bei dem zunächst eine Katalysatorvorstufe hergestellt wird, die aus Silber, Erdalkalimetallverbindung, insbesondere Calciumcarbonat, und der Promotorkombination aus der Cäsiumverbindung und einer oder mehrerer Verbindungen aus der Gruppe der Elemente Rhenium, Molybdän und Wolfram, besteht. Vorzugsweise wird die Vorstufe so hergestellt, dass Silber in Form eines löslichen Salzes oder Komplexes mit der Erdalkalimetallverbindung und der oben genannten Promotorkombination vermischt und anschließend calciniert wird. Die Calcinierung bewirkt die Umwandlung der Silberverbindung in elementares Silber. Auf diese Katalysatorvorstufe wird dann die Kaliumverbindung oder das Gemisch aus wenigstens zwei Verbindungen, von denen wenigstens eine eine Kaliumverbindung ist, aufgebracht, was beispielsweise durch Imprägnieren erfolgt.

Weiterer Gegenstand der Erfindung ist deshalb ein Verfahren zur Herstellung eines Katalysators zur Direktoxidation von Propylen mit Sauerstoff, umfassend die Stufen (α) bis (γ) durch
- (α): Vermischen von Silber in Form eines in Wasser gelösten Salzes oder Komplexes mit einem Trägermaterial, mindestens einer Erdalkalimetallverbindung und einer Kombination einer Cäsiumverbindung mit wenigstens einer Verbindung eines Elementes, ausgewählt aus der Gruppe Molybdän, Rhenium und Wolfram,
- (β): Calcinieren des erhaltenen feuchten oder vorgetrockneten Gemisches aus Stufe (α), wobei anschließend das erhaltene Material
- (γ): mit einer Lösung einer Kaliumverbindung oder eines Gemisches einer Kaliumverbindung mit mindestens einer weiteren Verbindung, wobei das Gemisch einen Schmelzpunkt unterhalb 340 °C aufweist, imprägniert wird.

In einer bevorzugten Ausführungsform der Erfindung wird der Katalysator in nachstehend beschriebener Weise hergestellt. Ethylendiamin und Oxalsäure werden zunächst in Wasser gelöst und dann zu dieser Lösung Silber(I)-oxid und Ethanolamin zugegeben. Nachdem sich das Silber(I)-oxid aufgelöst hat, werden wässrige Lösungen aus Cäsiumnitrat, Ammoniumperrhenat, Ammoniummolybdat und/oder Ammoniumwolframat sowie als Erdalkalimetallverbindung Calciumcarbonat in Form eines Pulvers zugegeben und die erhaltene Suspension vermischt. In die Suspension kann weiteres Trägermaterial, beispielsweise inertes Trägermaterial, eingerührt werden. Das feuchte Material wird dann etwa bei 60 °C im Vakuumtrockenschrank vorgetrocknet und dann calciniert, beispielsweise bei einer Temperatur von 100 bis 400 °C. Als Trocknungsverfahren können auch Sprühtrocknung, Sprühgranulationstrocknung sowie Zentrifugation eingesetzt werden. Nach dem Erkalten wird mit einer wässerigen Lösung aus Kaliumnitrat und der weiteren Verbindung, vorzugsweise Natriumnitrat, imprägniert und der erhaltene Katalysator anschließend getrocknet.

In einer besonders bevorzugten Ausführung ist demzufolge die Herstellung des erfindungsgemäßen Katalysators dadurch gekennzeichnet, dass zu einer wässerigen Lösung von Silber(I)-oxid, Ethanolamin, Ethylendiamin und Oxalsäure wässerige Lösungen von Cäsiumnitrat, Ammoniumperrhenat, Ammoniummolybdat und Ammoniumwolframat zugegeben werden und die erhaltene Suspension mit dem Trägermaterial, umfassend Calciumcarbonat, versetzt wird, das erhaltene feuchte Material getrocknet und bei einer Temperatur von 100 bis 400 °C calciniert wird und anschließend das Material mit einer wässerigen Lösung von Kaliumnitrat oder einer wässerigen Lösung von Kaliumnitrat und Natriumnitrat imprägniert wird.

Es ist auch möglich, das erhaltene Katalysatormaterial zu Formkörpern zu verarbeiten, die für den Einsatz in einem Reaktor geeignet sind. Geeignete Formkörper können zum Beispiel durch bekannte Methoden wie Tablettierung, Verstrangung oder Aufbauagglomeration hergestellt werden. Es ist dabei auch möglich, die Kaliumverbindung bzw. das Kalium enthaltende Promotorgemisch vor der Formgebung oder danach aufzubringen.

Die Form des Katalysators unterliegt keinen Beschränkungen. Er kann in jeder beliebigen Form ausgebildet sein. Vorzugsweise ist er in einer Form ausgebildet, die sich zum Einsatz in Festbett-, Fließbett- oder Wirbelbett-Reaktoren eignet.

Beispielsweise kann die Formgebung in konventionellen Extrudern durchgeführt werden. Hierbei können dann Extrudate erhalten werden, die beispielsweise einen Durchmesser von 1 bis 10 mm, insbesondere von 2 bis 5 mm besitzen. Solche Extrudate sind besonders gut für den Einsatz in vorstehend genannten Reaktoren geeignet.

Somit ist der erfindungsgemäße Katalysator und auch das Verfahren zu seiner Herstellung dadurch gekennzeichnet, dass die Katalysatoren als Körper ausgebildet werden, die sich zum Einsatz in Festbett-, Fließbett- oder Wirbelbettreaktoren eigenen.

Die Erfindung wird anhand der folgenden Beispiele näher erläutert.

### Beispiele

### Beispiel 1: Herstellung eines Katalysators

Es wurden 100 g Wasser mit 101,7 g Ethylendiamin vermischt und darin 103,8 g Oxalsäuredihydrat unter Rühren gelöst. Zu der Lösung wurden portionsweise 181,6 g Silber(I)-oxid sowie 38,3 g Ethanolamin zugegeben. Nachdem sich das Silber(I)-oxid vollständig gelöst hatte, wurde mit Wasser auf 500 ml Gesamtvolumen aufgefüllt. Zu 87 g dieser Lösung wurden 0,63 g Ammoniumheptamolybdat-tetrahydrat, 0,87 g Ammoniumperrhenat, 0,041 g Cäsiumnitrat (CsNO₃) sowie 16,2 g Calciumcarbonat (CaCO₃) hinzugegeben. Die Mischung wurde mit einem Dispergierapparat (Ultra-Turrax) so lange intensiv vermischt, bis eine homogene Suspension entstanden war. In diese Suspension wurden 28,2 g eines α-Aluminiumoxids (Fa. Norton, SA 5262), die in Form einer Splittfraktion vorlag (Teilchendurchmesser ca. 0,6 bis 1,6 mm), eingerührt. Die feuchte Katalysatorvorstufe wurde in einem Vakuumtrockenschrank bei 60 °C für 20 Stunden getrocknet und dann in einem Drehrohrofen unter Luft bei 300 °C für drei Stunden calciniert.

Von der erhaltenen Katalysatorvorstufe wurden 20,5 g mit einer Lösung aus 0,82 g Kaliumnitrat und 0,21 g Natriumnitrat in 10 ml Wasser imprägniert und anschließend bei 100 °C getrocknet, wobei der Katalysator erhalten wurde.

### Beispiel 2:

Es wurden 100 g Wasser mit 101,7 g Ethylendiamin vermischt und darin 103,8 g Oxalsäuredihydrat unter Rühren gelöst. Zu der Lösung wurden 181,6 g Silber(I)-oxid sowie 38,3 g Ethanolamin zugegeben. Nachdem sich das Silber(I)-oxid vollständig gelöst hatte, wurde mit Wasser auf 500 ml Gesamtvolumen aufgefüllt. Zu 67,3 g dieser Lösung wurden 0,6 g Ammoniumheptamolybdat-tetrahydrat, 0,027 g Cäsiumnitrat sowie 16,2 g Calciumnitrat hinzugegeben. Die Suspension wurde mit einem Dispergierapparat (Ultra-Turrax) so lange intensiv vermischt, bis eine homogene Suspension entstanden war. In diese Mischung wurden 28,2 g eines α-Aluminiumoxids (Norton, SA 5262) in Form einer Splittfraktion (Teilchendurchmesser ca. 0,6 bis 1,6 mm) eingerührt. Der feuchte Träger wurde in einem Vakuumtrockenschrank bei 60 °C für 20 Stunden getrocknet und dann in einem Drehrohrofen unter Luft bei 300 °C für drei Stunden calciniert.

Von der erhaltenen Vorstufe des Katalysators wurden 20,5 g mit einer Lösung aus 0,82 g Kaliumnitrat und 0,21 g Natriumnitrat in 10 ml Wasser imprägniert und anschließend bei 100 °C getrocknet, wobei der Katalysator erhalten wurde.

### Beispiel 3:

Es wurde verfahren wie in Beispiel 2 angegeben, mit dem Unterschied, dass statt 0,027 g Cäsiumnitrat 0,041 g Cäsiumnitrat eingesetzt wurden.

### Beispiel 4:

Es wurden 100 g Wasser mit 101,7 g Ethylendiamin vermischt und darin 103,8 g Oxalsäuredihydrat unter Rühren gelöst. Zu der Lösung wurden 181,6 g Silber(I)-oxid sowie 38,3 g Ethanolamin zugegeben. Nachdem sich das Silber(I)-oxid vollständig gelöst hatte, wurde mit Wasser auf 500 ml Gesamtvolumen aufgefüllt. Zu 67,3 g dieser Lösung wurden 0,44 g Ammoniumperrhenat, 0,018 g CsNO₃ sowie 16,2 g CaCO₃ hinzugegeben. Die Suspension wurde mit einem Dispergierapparat (Ultra-Turrax) so lange intensiv vermischt, bis eine homogene Suspension entstanden war. In diese Mischung wurden 28,2 g eines α-Aluminiumoxids (Norton, SA 5262) in Form einer Splittfraktion (Teilchendurchmesser ca. 0,6 bis 1,6 mm) eingerührt. Der feuchte Träger wurde in einem Vakuumtrockenschrank bei 60 °C für 20 Stunden getrocknet und dann in einem Drehrohrofen unter Luft bei 300 °C für drei Stunden calciniert.

Von der erhaltenen Katalysatorvorstufe wurden 20,5 g mit einer Lösung aus 0,82 g Kaliumnitrat und 0,21 g Natriumnitrat in 10 ml Wasser imprägniert und anschließend bei 100 °C getrocknet, wobei der Katalysator erhalten wurde.

### Beispiel 5:

Es wurde verfahren wie in Beispiel 4, mit dem Unterschied, dass statt 0,44 g Ammoniumperrhenat 0,87 g Ammoniumperrhenat eingesetzt wurden.

### Beispiel 6:

Beispiel 4 wurde wiederholt mit dem Unterschied, dass statt 0,44 g Ammoniumperrhenat 1,3 g Ammoniumperrhenat eingesetzt wurden.

### Beispiel 7:

Es wurden 100 g Wasser mit 101,7 g Ethylendiamin vermischt und darin 103,8 g Oxalsäuredihydrat unter Rühren gelöst. Zu der Lösung wurden 181,6 g Silber(I)-oxid sowie 38,3 g Ethanolamin zugegeben. Nachdem sich das Silber(I)-oxid vollständig gelöst hatte, wurde mit Wasser auf 500 ml Gesamtvolumen aufgefüllt. Zu 67,3 g dieser Lösung wurden 0,6 g Ammoniumheptamolybdat-tetrahydrat, 0,87 g Ammoniumperrhenat, 0,027 g Cäsiumnitrat sowie 16,2 g Calciumcarbonat hinzugegeben. Die Suspension wurde mit einem Dispergierapparat (Ultra-Turrax) so lange intensiv vermischt, bis eine homogene Suspension entstanden war. In diese Mischung wurden 28,2 g eines α-Aluminiumoxids (Norton, SA 5262) in Form einer Splittfraktion (Teilchendurchmesser ca. 0,6 bis 1,6 mm) eingerührt. Der feuchte Träger wurde in einem Vakuumtrockenschrank bei 60 °C für 20 Stunden getrocknet und dann in einem Drehrohrofen unter Luft bei 300 °C für drei Stunden calciniert.

Von der erhaltenen Vorstufe des Katalysators wurden 20,5 g mit einer Lösung aus 0,82 g Kaliumnitrat und 0,21 g Natriumnitrat in 10 ml Wasser imprägniert und anschließend bei 100 °C getrocknet, wobei der Katalysator erhalten wurde.

### Beispiel 8:

Es wurde verfahren wie in Beispiel 7 mit dem Unterschied, dass statt 0,027 g Cäsiumnitrat 0,041 g Cäsiumnitrat eingesetzt wurden.

### Beispiel 9:

Es wurden 100 g Wasser mit 101,7 g Ethylendiamin vermischt und darin 103,8 g Oxalsäuredihydrat unter Rühren gelöst. Zu der Lösung wurden 181,6 g Silber(I)-oxid sowie 38,3 g Ethanolamin zugegeben. Nachdem sich das Silber(I)-oxid vollständig gelöst hatte, wurde mit Wasser auf 500 ml Gesamtvolumen aufgefüllt. Zu 67,3 g dieser Lösung wurden 0,6 g Ammoniumheptamolybdat-tetrahydrat, 0,87 g Ammoniumperrhenat, 0,018 g CsNO₃, 0,005 g (NH₄)SO₄ sowie 16,2 g CaCO₃ hinzugegeben. Die Suspension wurde mit einem Dispergierapparat (Ultra-Turrax) so lange intensiv vermischt, bis eine homogene Suspension entstanden war. In diese Mischung wurden 28,2 g eines α-Aluminiumoxids (Norton, SA 5262) in Form einer Splittfraktion (Teilchendurchmesser ca. 0,6 bis 1,6 mm) eingerührt. Der feuchte Träger wurde in einem Vakuumtrockenschrank bei 60 °C für 20 Stunden getrocknet und dann in einem Drehrohrofen unter Luft bei 300 °C für drei Stunden calciniert.

Von der erhaltenen Vorstufe des Katalysators wurden 20,5 g mit einer Lösung aus 0,82 g Kaliumnitrat und 0,21 g Natriumnitrat in 10 ml Wasser imprägniert und anschließend bei 100 °C getrocknet, wobei der Katalysator erhalten wurde.

### Vergleichsbeispiel 1:

Es wurde verfahren wie in Beispiel 2 mit dem Unterschied, dass kein Cäsiumnitrat und kein Natriumnitrat zugegeben wurden.

### Vergleichsbeispiel 2:

Es wurde verfahren wie in Beispiel 4 mit dem Unterschied, dass kein Cäsiumnitrat und kein Natriumnitrat zugegeben wurden.

### Vergleichsbeispiel 3:

Es wurde verfahren wie in Beispiel 2 mit dem Unterschied, dass kein Cäsiumnitrat zugegeben wurde.

### Vergleichsbeispiel 4:

Es wurde verfahren wie in Beispiel 7 mit dem Unterschied, dass kein Cäsiumnitrat zugegeben wurde.

### Durchführung der Direktoxidation von Propylen zu Propylenoxid

Eine Apparatur, bestehend aus einem mantelbeheizten Edelstahlrohr von 400 mm Länge und 8 mm Innendurchmesser wurde mit 20 ml der in den Beispielen 1 bis 9 und den Vergleichsbeispielen 1 bis 4 beschriebenen Katalysatoren beschickt.

In einer Versuchsausführung (Testbedingung A) wurden 86,6 ml/min eines Gasgemisches, bestehend aus 5,3 Vol.-% Propylen, 17,6 Vol.-% Sauerstoff, 45 ppm Stickoxid (Stickstoffmonoxid und/oder Stickstoffdioxid) sowie 220 ppm Ethylchlorid, bei einer Temperatur von 230 °C und einem Druck von 3 bar durch das Katalysatorbett geleitet. Der restliche Anteil des Gasgemischs bestand aus Stickstoff.

In einer weiteren Versuchsausführug (Testbedingung B) enthielt das Gasgemisch 12 Vol.-% Propylen, 24,5 Vol.-% Sauerstoff, 40 ppm Stickoxid (Stickstoffmonoxid und/oder Stickstoffdioxid) sowie 200 ppm Ethylchlorid. Der restliche Anteil des Gasgemischs bestand aus Stickstoff. Das Gasgemisch wurde bei einer Temperatur von 220 °C und einem Druck von 3 bar durch das Katalysatorbett geleitet.

Das Abgas wurde mittels eines online geschalteten Gaschromatographen analysiert. Die Ergebnisse nach 50 bis 150 Betriebsstunden sind in den Tabellen 1 bis 3 angegeben.

Die Ergebnisse aus Tabelle 1 wurden unter Testbedingung A erzielt. Der in Beispiel 9 eingesetzte Katalysator wies im Gegensatz zu den Katalysatoren aus Beispiel 1 bis 8 zusätzlich einen Schwefelgehalt von 20 ppm auf. Die Katalysatoren wiesen zudem alle einen Gehalt an Natriumnitrat von 1 Gew.-% auf.

Die Ergebnisse aus Tabelle 2 wurden unter Testbedingung B erzielt. Der Katalysator wies einen Gehalt an Natriumnitrat von 1 Gew.-% auf.

Für die Herstellung der Katalysatoren der Vergleichsbeispiele 1 bis 4 wurde keine Cäsiumverbindung verwendet. Dabei enthielten die Katalysatoren der Vergleichsbeispiele 1 und 2 kein Natriumnitrat, die Katalysatoren der Vergleichsbeispiele 2 und 3 hingegen jeweils 1 Gew.-% Natriumnitrat. Die mit den Katalysatoren der Vergleichsbeispiele erzielten Ergebnisse der Tabelle 3 wurden unter der Testbedingung A erreicht.

Die Propylenoxid-Selektivität S_{PO} wurde nach der Formel S_{PO} = C_{PO} / (C_{PO} + 1/3 C_{CO2}) berechnet, wobei C_{PO} und C_{CO2} die Konzentrationen an Propylenoxid bzw. Kohlendioxid im Abgas angeben, jeweils bezogen auf 100 Vol.-% Abgas.
Alle Angaben in ppm beziehen sich auf das Gewicht.

**Tabelle 1**

| Katalysator aus Beispiel | Mo - Gehalt [Gew.-%] | Re - Gehalt [Gew.-%] | Cs - Gehalt [ppm] | S_{PO} [Vol.-%] | C_{PO} [Vol.-%] |
|---|---|---|---|---|---|
| 1 | 0,5 | 1,0 | 450 | 56 | 1,0 |
| 2 | 0,5 | 0 | 300 | 58 | 0,8 |
| 3 | 0,5 | 0 | 450 | 58 | 1,0 |
| 4 | 0 | 0,5 | 200 | 54 | 1,3 |
| 5 | 0 | 1,0 | 200 | 58 | 0,8 |
| 6 | 0 | 1,5 | 200 | 58 | 0,9 |
| 7 | 0,5 | 1,0 | 300 | 58 | 0,9 |
| 8 | 0,5 | 1,0 | 450 | 60 | 0,8 |
| 9 | 0,5 | 1,0 | 200 | 60 | 0,9 |

**Tabelle 2**

| Katalysator aus Beispiel | Mo - Gehalt [Gew.-%] | Re - Gehalt [Gew.-%] | Cs - Gehalt [ppm] | S_{PO} [Vol.-%] | C_{PO} [Vol.-%] |
|---|---|---|---|---|---|
| 1 | 0,5 | 1,0 | 450 | 62 | 1,2 |

**Tabelle 3**

| Katalysator aus Vergleichsbeispiel | Mo - Gehalt [Gew.-%] | Re - Gehalt [Gew.-%] | Cs - Gehalt [ppm] | S_{PO} [Vol.-%] | C_{PO} [Vol.-%] |
|---|---|---|---|---|---|
| 1 | 0,5 | 0 | 0 | 40 | 0,9 |
| 2 | 0 | 0,5 | 0 | 42 | 1,1 |
| 3 | 0,5 | 0 | 0 | 54 | 1,1 |
| 4 | 0,5 | 1,0 | 0 | 49 | 0,8 |

Wie den Tabellen zu entnehmen ist, lagen die mit den erfindungsgemäßen Katalysatoren erzielten Selektivitäten durchweg höher als mit den Vergleichskatalysatoren. Beispiel 4 erreichte zwar nur die Selektivität des Vergleichsbeispiels 3, jedoch war die erzielte Ausbeute an Propylenoxid deutlich höher. Insgesamt ergab sich ein sehr ausgewogenes Verhältnis von erreichter Selektivität zu Propylenoxid-Ausbeute.

Mit dem Katalysator aus Beispiel 1 der Tabelle 2 wurden unter den Testbedingungen B überlegene Eigenschaften erreicht.

## Patentansprüche

1. Verfahren zur Herstellung von Propylenoxid durch Gasphasenoxidation eines Gemisches, umfassend Propylen und Sauerstoff, bei einer Temperatur von 100 bis 300 °C und einem Druck von 1 bis 30 bar an einem Katalysator, **dadurch gekennzeichnet, dass** der Katalysator
(i) Silber,
(ii) mindestens eine Erdalkalimetallverbindung,
(iii) eine Kaliumverbindung oder ein Gemisch einer Kaliumverbindung mit mindestens einer weiteren Verbindung, wobei das Gemisch einen Schmelzpunkt unterhalb 340 °C aufweist, und
(iv) eine Kombination einer Cäsiumverbindung mit wenigstens einer Verbindung eines Elementes, ausgewählt aus der Gruppe Molybdän, Rhenium und Wolfram,
umfasst.

2. Verfahren nach Anspruch 1, **dadurch gekennzeichnet, dass** die Erdalkalimetallverbindung ausgewählt wird aus der Gruppe bestehend aus Calcium-, Strontium-, Bariumcarbonat und einem Gemisch aus zwei oder mehr davon.

3. Verfahren nach Anspruch 1 oder 2, **dadurch gekennzeichnet, dass** das Trägermaterial Aluminiumoxid, Siliciumdioxid, Siliciumcarbid, Titandioxid, Zirkoniumdioxid oder ein Gemisch aus zwei oder mehr davon umfasst.

4. Verfahren nach einem der Ansprüche 1 bis 3, **dadurch gekennzeichnet, dass** die Kaliumverbindung Kaliumnitrat ist oder ein Gemisch aus Kaliumnitrat mit mindestens einer Verbindung, ausgewählt aus der Gruppe bestehend aus Natriumnitrat, Natriumnitrit, Lithiumnitrat, Bleinitrat, Bariumnitrat, Silbernitrat, Cäsiumnitrat, Rubidiumnitrat und Thalliumnitrat.

5. Verfahren nach einem der Ansprüche 1 bis 4, **dadurch gekennzeichnet, dass** die Cäsiumverbindung Cäsiumnitrat, Cäsiumhydroxid, Cäsiumchlorid, Cäsiumfluorid, Cäsiumsulfat, Cäsiumoxalat oder Cäsiumacetat ist, und die Verbindungen der Metalle Rhenium, Molybdän und Wolfram Ammoniumpherrhenat, Rhenium(III)-chlorid, Rhenium(V)-chlorid, Rhenium(VI)-fluorid, Rhenium(VI)-oxid, Rhenium(VII)-oxid, Ammoniumwolframat, Ammoniummetawolframat, Wolfram(IV)-chlorid, Wolfram(VI)-chlorid, Wolframoxytetrachlorid, Wolframsäure, Ammoniummolybdat, Molybdän(III)-chlorid, Molybdän(V)-chlorid und Molybdän(VI)-chlorid-oxid sind.

6. Verfahren nach Anspruch 5, **dadurch gekennzeichnet, dass** die Cäsiumverbindung Cäsiumnitrat oder Cäsiumhydroxid ist und kombiniert wird mit einer oder mehreren Verbindungen ausgewählt aus der Gruppe Ammoniumperrhenat, Ammoniumwolframat und Ammoniummolybdat.

7. Katalysator zur Direktoxidation von Propylen mit Sauerstoff, **dadurch gekennzeichnet, dass** der Katalysator
(i) Silber,
(ii) mindestens eine Erdalkalimetallverbindung,
(iii) eine Kaliumverbindung oder ein Gemisch einer Kaliumverbindung mit mindestens einer weiteren Verbindung, wobei das Gemisch einen Schmelzpunkt unterhalb 340 °C aufweist, und
(iv) eine Kombination einer Cäsiumverbindung mit wenigstens einer Verbindung mit einem Element ausgewählt aus der Gruppe Molybdän, Rhenium und Wolfram
umfasst.

8. Verfahren zur Herstellung des Katalysators gemäß Anspruch 7, umfassend die Stufen (α) bis (γ) durch
(α) Vermischen von Silber in Form eines in Wasser gelösten Salzes oder Komplexes mit einem Trägermaterial, mindestens einer Erdalkalimetallverbindung und einer Kombination einer Cäsiumverbindung mit wenigstens einer Verbindung eines Elementes ausgewählt aus der Gruppe Molybdän, Rhenium und Wolfram,
(β) Calcinieren des erhaltenen feuchten oder vorgetrockneten Gemisches aus (α), wobei anschließend das erhaltene Material
(γ) mit einer Lösung einer Kaliumverbindung oder eines Gemisches einer Kaliumverbindung mit mindestens einer weiteren Verbindung, wobei das Gemisch einen Schmelzpunkt unterhalb 340 °C aufweist, imprägniert wird.

9. Verfahren nach Anspruch 8, **dadurch gekennzeichnet, dass** zu einer wässerigen Lösung von Silber(I)-oxid, Ethanolamin, Ethylendiamin und Oxalsäure, wässerige Lösungen von Cäsiumnitrat, Ammoniumperrhenat, Ammoniummolybdat und Ammoniumwolframat zugegeben und die erhaltene Suspension mit einem Trägermaterial umfassend Calciumcarbonat versetzt, das erhaltene feuchte Material getrocknet und bei einer Temperatur von 100 bis 400 °C calciniert und anschließend der Körper mit einer wässerigen Lösung von Kaliumnitrat oder einer wässerigen Lösung von Kaliumnitrat und Natriumnitrat imprägniert wird.

10. Verfahren nach Anspruch 8 oder Anspruch 9, **dadurch gekennzeichnet, dass** die Katalysatoren als Körper ausgebildet werden, die sich zum Einsatz in Festbett-, Fließbett- oder Wirbelbettreaktoren eignen.
